# EUROPÄISCHE PATENTANMELDUNG

(11) **EP 1 884 563 A1**
(43) Veröffentlichungstag der Anmeldung: **06.02.2008**
(21) Anmeldenummer: 07102358.4
(22) Anmeldetag: 14.02.2007
(51) Int. Cl.: C12M 1/33

(54) **Vorrichtung zur Erzeugung einer Suspension aus Biomasse**

(30) Priorität: 01.08.2006 DE 202006011872 U
(71) Anmelder: Eckard, Horst K., 36169 Rasdorf (DE); Grimm, Arnold, 36154 Hosenfeld (DE); Hemschemeier, Hans, 51647 Gummersbach (DE)
(72) Erfinder: Eckard, Horst K., 36169 Rasdorf (DE); Grimm, Arnold, 36154 Hosenfeld (DE); Hemschemeier, Hans, 51647 Gummersbach (DE)
(74) Vertreter: Schwabe - Sandmair - Marx

(57) **Zusammenfassung**

Vorrichtung zur Erzeugung einer Suspension aus Biomasse, die Vorrichtung umfassend:
a) einen Einlass (3, 6) für die Biomasse,
b) einen Zerkleinerer (4) oder Homogenisierer (11) für die Zerkleinerung der Biomasse,
c) einen stromab von dem Zerkleinerer (4) oder Homogenisierer (11) angeordneten Auslass (12) für die Biomasse,
d) einen Behälter (7, 10) für die Biomasse,
e) eine Fördereinrichtung (4, 5, 8) für die Biomasse,
f) und eine Plattform (1), auf welcher der Einlass (3, 6), der Auslass (12), der Zerkleinerer (4) oder Homogenisierer (11), der Behälter (7, 10) und die Fördereinrichtung (4, 5, 8) angeordnet sind,
g) wobei die Vorrichtung als Einheit auf der Straße oder Schiene transportabel ist.

## Beschreibung

Die Erfindung betrifft die Zusammenfassung einzelner technischer Komponenten für die Erzeugung von Suspensionen aus Biomasse für die Biogas-Erzeugung. Installiert sind diese technischen Komponenten auf einer transportablen gemeinsamen Plattform, vorzugsweise in einem Container.

Durch mikrobiellen Abbau wird in einem Fermenter einer Biogasanlage methanhaltiges Biogas erzeugt, das in einem dem Fermenter nachgeschalteten Anlagenteil für die weitere Verwendung aufbereitet wird, beispielsweise für die Verbrennung in einem Generator zur Stromerzeugung. Die zur Anlage gelieferten Biomassen werden in einem dem Fermenter vorgeschalteten Anlagenteil aufbereitet, um den mikrobiellen Abbau zu fördern oder überhaupt erst zu ermöglichen. Dieser Aufbereitungsteil umfasst Einlässe für die Zuführung unterschiedlicher Biomassen, eine Mischeinrichtung und einen Zerkleinerer zum Mischen der Biomassen und Zerkleinern fester Bestandteile. Die Komponenten des Aufbereitungsteils, insbesondere die Mischeinrichtung, der Zerkleinerer, eine Fördereinrichtung, die Anschlüsse, Ventile und Leitungen, werden für die jeweilige Biogasanlage individuell zusammengestellt und vor Ort installiert. Anschließend werden die Komponenten im System aufeinander abgestimmt, bis die Anlage schließlich in Betrieb genommen werden kann. Dies führt zu hohen Kosten. Die Einhaltung von Mindeststandards, insbesondere Sicherheitsstandards, ist in vielen Fällen nicht gewährleistet, zumindest wird der Nachweis für einen ordnungsgemäßen, den Bestimmungen entsprechenden Betrieb nur in seltenen Fällen erbracht. Der Nachweis ist auch mit beachtlichen Kosten verbunden.

Es ist eine Aufgabe der Erfindung, die Kosten für die Errichtung einer Biogasanlage zu verringern.

Gelöst wird die Aufgabe mittels einer Vorrichtung, die mehrere Komponenten, die einem Fermenter einer Biogasanlage vorgeschaltet sind oder erst noch vorgeschaltet werden, auf einer Plattform zusammenfasst, so dass die gesamte Vorrichtung als Einheit transportabel ist. Die in diesem Sinne mobile Vorrichtung weist im Ganzen Abmessungen auf, die den Transport auf der Straße oder der Schiene ermöglichen. Die Plattform ist vorzugsweise so gestaltet, dass sie auf üblichen Sattelschleppern und Güterwaggons transportiert werden kann.

Auf der Plattform sind wenigstens ein Einlass für Biomasse, vorzugsweise für noch nicht behandelte Rohmasse, und wenigstens ein Auslass für die auf der Plattform behandelte Biomasse angeordnet. Ferner sind auf der Plattform ein Zerkleinerer oder Homogenisierer für die Zerkleinerung oder Homogenisierung der durch den Einlass zuführbaren Biomasse, eine Fördereinrichtung und ein Behälter für die Biomasse installiert. Der Behälter kann der Aufnahme der gesamten auf der Plattform behandelbaren Biomasse oder nur eines Teils der Biomasse dienen. Für den Zerkleinerer oder Homogenisierer gilt, dass in bevorzugten Ausführungen zwar die gesamte auf der Plattform behandelbare Biomasse durch den Zerkleinerer oder Homogenisierer führbar ist, dass aber auch ein Aufbereitungssystem für eine Biogasanlage Gegenstand der Erfindung ist, bei dem nur ein Teil der auf der Plattform behandelbaren Biomasse durch den Zerkleinerer oder Homogenisierer geführt wird oder geführt werden kann.

Das Wort "oder" wird im üblichen logischen Sinne von "und/oder" verstanden, soweit im jeweiligen Zusammenhang nicht auf eine Bedeutung im Sinne eines exklusiven "oder" hingewiesen wird oder eine solche Bedeutung nur gemeint sein kann.

In einer vorteilhaften Weiterbildung ist die Plattform ein Container und wird im Folgenden daher auch als Container-Plattform bezeichnet. Die Container-Plattform umgibt abgesehen von Anschlüssen für die Zuführung der zu behandelnden Biomasse und die Abführung der erzeugten Suspension sowie optional weiteren Anschlüssen für beispielsweise Biomasse, Wasser oder für eine oder mehrere im Kreislauf zurückgeführte Fraktionen die installierten Komponenten. Die Container-Plattform ist vorzugsweise zumindest im Wesentlichen ein Quader. Sie kann nach einer einzigen oder auch nach mehreren Seiten offen sein, beispielsweise nach oben. Vorzugsweise ist sie allseitig geschlossen. Sie ist vorteilhafterweise für Inspektionen und Einstell-, Wartungs- und Reparaturarbeiten begehbar.

Die Vorrichtung, d.h. die Plattform mit den darauf angeordneten Komponenten, weist in bevorzugten Ausführungen über alles gesehen eine Breite von höchstens 2,5 m und eine Höhe von höchstens 4 m, bevorzugter höchstens 3 m auf, d.h. ein die Vorrichtung umschreibender Quader hat eine Breite von höchstens 2,5 m und eine Höhe von höchstens 4 m in solchen Ausführungen. Vorzugsweise betragen sowohl die Breite als auch die Höhe höchstens 2,5 m.

Die Erfindung ermöglicht nicht nur den einfachen Transport einer vormontierten Vorrichtung zum Ort der Biogasanlage, sondern vereinfacht auch die Aufstellung vor Ort. Es wird auch die für die Errichtung der Anlage erforderliche Zeit verkürzt. Insbesondere kann die im Ganzen ab Werk lieferbare Vorrichtung einmalig ein Zulassungsverfahren durchlaufen und nach Zulassung in Serie gefertigt und ausgeliefert werden, so dass sich das Zulassungsverfahren für die mit der Vorrichtung ausgerüstete Biogasanlage erheblich vereinfacht und verkürzt. Alle diese Faktoren tragen zur Reduzierung der Kosten für die Biogasanlage und die Erhöhung der Betriebssicherheit bei.

Die Erfindung betrifft daher auch ein Verfahren zur Herstellung von Vorrichtungen zur Aufbereitung von Biomasse für den mikrobiellen Abbau in Fermentern von Biogasanlagen. Das Verfahren zeichnet sich dadurch aus, dass eine erfindungsgemäße Vorrichtung eine Zertifizierung durchläuft, um den Nachweis eines einwandfreien und den einschlägigen Richtlinien, insbesondere Sicherheits- und Umweltrichtlinien, gerechten Betriebs ihrer Komponenten im Zusammenwirken zu führen. Nach der Zertifizierung wird die Vorrichtung in stets gleicher Ausführung hergestellt, vorzugsweise in Serie, d.h. in Kleinserie, wobei die weiteren Vorrichtungen der einmalig zertifizierten Vorrichtung soweit entsprechen, dass deren Zertifizierung für die weiteren Vorrichtungen gilt.

Für die Komponenten der Vorrichtung oder einen Teil der Komponenten werden vorteilhafterweise jeweils am Markt bereits erhältliche und bewährte technische Einrichtungen verwendet.

Obgleich die Erfindung grundsätzlich nur fordert, dass auf der Plattform entweder der Zerkleinerer oder der Homogenisierer angeordnet ist, entspricht es bevorzugten Ausführungen, wenn auf der Plattform sowohl der Zerkleinerer als auch der Homogenisierer angeordnet sind. Der Zerkleinerer und der Homogenisierer unterscheiden sich im Ergebnis der jeweiligen Behandlung. Die vom Homogenisierer behandelte Biomasse weist einen höheren Zerkleinerungsgrad als die vom Zerkleinerer behandelte Biomasse auf. Allerdings wird dem Homogenisierer vorteilhafterweise nur Biomasse mit größten Festbestandteilen zugeführt, die kleiner sind als die größten Festbestandteile in der dem Zerkleinerer zugeführten Biomasse. Der Homogenisierer ist dem Zerkleinerer in bevorzugten Ausführungen nachgeschaltet, so dass die bereits im Zerkleinerer zerkleinerte Biomasse, nämlich die gesamte oder nur ein Teil der im Zerkleinerer zerkleinerten Biomasse, im Homogenisierer feiner zerkleinert wird. Vorzugsweise ist der Homogenisierer dazu eingerichtet, die ihm zugeführte Biomasse soweit zu zerkleinern, dass den Homogenisierer eine homogene, fließfähige Biomasse mit größten Partikeln von höchsten 4 mm, vorzugsweise höchstens 2 mm, verlässt. Noch bevorzugter ist der Homogenisierer so ausgebildet, dass die größten Partikel am Auslass des Homogenisierers eine maximale Erstreckung von höchstens 100 Mikrometern oder wenigen hundert Mikrometern haben. In den Homogenisierer wird vorzugsweise eine Biomasse eingebracht, die eine Mischung mit festen biologischen Bestandteilen und einer Flüssigkeit ist. Die mittels des Homogenisierers dispergierte Biomasse weist vorteilhafterweise eine sahneförmige Konsistenz auf, d.h. sie ist emulsionsartig. Mit dem Homogenisierer gelingt insbesondere die in herkömmlichen Aufbereitungsanlagen problematische Zerkleinerung von zellulosehaltigem Fasermaterial auf maximale Längen aus dem unteren Millimeterbereich bis in den Submillimeterbereich.

Der Homogenisierer umfasst wenigstens eine Zerkleinerungsstufe aus einem ersten Scherkranz, einem zweiten Scherkranz und einem Drehantrieb für den ersten Scherkranz. Der erste Scherkranz bildet einen Rotor. Der zweite Scherkranz bildet einen Stator, der vorzugsweise still steht und verdrehgesichert mit einem Gehäuse des Homogenisierers verbunden ist. Grundsätzlich könnte jedoch zusätzlich auch der zweite Scherkranz relativ zu dem Gehäuse drehangetrieben sein. Der erste Scherkranz weist in Umfangsrichtung voneinander beabstandete Scherkanten auf, die quer zu der Umfangsrichtung des Scherkranzes weisen. Der zweite Scherkranz weist ebenfalls in Umfangsrichtung um die Rotationsachse voneinander beabstandete Scherkanten auf, die Schergegenkanten für die Scherkanten des ersten Scherkranzes bilden und quer zu der Umfangsrichtung des zweiten Scherkranzes weisen. Die Scherkanten und Schergegenkanten erstrecken sich vorzugsweise parallel zu der Rotationsachse des zweiten Scherkranzes. Grundsätzlich könnten Sie zu der Rotationsachse jedoch auch geneigt sein, solange sie nur eine quer zu der Umfangsrichtung weisende Richtungskomponente haben. Zwischen den beiden Scherkränzen der Zerkleinerungsstufe verbleibt um die Rotationsachse herum ein enger Spalt, in den die zu zerkleinernde Biomasse einleitbar ist. In dem Spalt werden die festen Bestandteile der eingebrachten Biomasse mittels der relativ zueinander rotierenden Scherkanten und Schergegenkanten zerkleinert, wobei die Zerkleinerung zumindest zu einem Teil auf einer Scherwirkung der relativ zueinander bewegten Scher- und Schergegenkanten beruht. Von besonderem Vorteil für die Zerkleinerung sind die Druckpulsationen die mit jedem Schervorgang einhergehen. Die Drehgeschwindigkeit, welche die beiden Scherkränze relativ zueinander aufweisen, ist vorteilhafterweise so groß, dass die festen Biobestandteile über die Scherwirkung hinaus auch durch Kavitation zerkleinert werden. Vorzugsweise ist die Geschwindigkeit so groß, dass die Kavitation an der Zerkleinerung einen in etwa ebenso großen oder vorteilhafterweise sogar größeren Anteil als die Scherwirkung hat. Um Kavitation in einem nennenswerten Ausmaß zu erzeugen, wird der Homogenisierer mit solch einer Geschwindigkeit drehangetrieben, dass radial nächstbenachbarte Scherkränze am Umfang eine Relativgeschwindigkeit von wenigstens 30 m/s, vorzugsweise wenigstens 50 m/s und noch bevorzugter wenigstens 60 m/s zueinander haben.

Die Biomasse könnte grundsätzlich zwar in axialer Richtung in den Spalt eingebracht werden, bevorzugter strömt sie jedoch durch einen der Scherkränze in den Spalt und in einer Weiterentwicklung durch den anderen der beiden Scherkränze auch wieder aus dem Spalt. In derartigen Ausführungen ist wenigstens einer der Scherkränze, vorzugsweise sind beide Scherkränze, radial zur Rotationsachse durchlässig für die Biomasse. Grundsätzlich würde es hierfür genügen, wenn nur zwischen zwei benachbarten Scherkanten oder Schergegenkanten im jeweiligen Scherkranz ein Durchlass geformt ist, bevorzugter ist ein Durchlass jedoch zwischen je zwei nächstbenachbarten Scherkanten oder Schergegenkanten vorhanden.

Der Homogenisierer ist vorteilhaft zwar insbesondere in Kombination mit der beanspruchten Erfindung, nämlich der Anordnung von Komponenten eines Aufbereitungsteils für eine Biogasanlage auf einer gemeinsamen Plattform, er ist jedoch auch als solcher vorteilhaft für die Aufbereitung von Biomasse, insbesondere einer Dispersion fester Biobestandteile in einer Flüssigkeit, wie beispielsweise faseriger Festbestandteile in Gülle, Wasser oder einem Gemisch derartiger Flüssigkeiten.

Die Biomasse enthält nachwachsende Rohstoffe aus land- oder forstwirtschaftlicher Produktion. Sie kann pflanzliche oder tierische Abfallprodukte enthalten, auch Speisereste oder Schlachtabfälle. Sie kann frische biologische Produkte, beispielsweise Gras, Futter- oder Energiepflanzen, oder Silage, Heu oder Stroh enthalten. Klärschlamm kann ebenfalls beigemischt werden. Die Ausgangsstoffe oder -fraktionen können einzeln oder vorteilhafterweise auch in beliebiger Kombination Inhaltsstoffe der Biomasse sein, die mittels der Vorrichtung aufbereitet wird. Aufgrund der in bevorzugten Ausführungen feinen Dispergierung der Festbestandteile wird die Geschwindigkeit der Vergärung im Fermenter erhöht und die erforderliche Verweildauer verringert. Sind die Feststoffpartikel bis auf eine maximale Erstreckung von höchstens 2 mm oder vorzugsweise noch weiter zerkleinert, nähern sich die Kennlinien der Vergärung unterschiedlichster Pflanzen, beispielsweise von frischem Mais und Gras, einander an. Gemeint sind die Kennlinien m(t), d. h. die aus dem jeweiligen Inhaltsstoff, also einer bestimmten Pflanzensorte in einem bestimmten Zustand (beispielsweise frisch, getrocknet oder siliert) durch Vergärung erzeugte Gasmenge über der Verweildauer im Fermenter. Andererseits dienen die nach wie vor vorhandenen kleinen, gleichmäßig fein verteilten Feststoffpartikel den Mikroorganismen im Fermenter als Halt, so dass in den Fermenter keine zusätzlichen Stützstrukturen für die Mikroorganismen eingebracht werden müssen.

Der Homogenisierer umfasst in bevorzugten Weiterentwicklungen wenigstens eine, bevorzugter mehrere, weitere Zerkleinerungsstufen. Die Biomasse wird sukzessive durch die mehreren Zerkleinerungsstufen gefördert und von Stufe zu Stufe feiner zerkleinert. Die Serienschaltung kann dergestalt sein, dass die mehreren Zerkleinerungsstufen in Bezug auf die Rotationsachse entweder nur radial oder nur axial hintereinander angeordnet sind. Bevorzugter sind wenigstens zwei Zerkleinerungsstufen in der Art der erläuterten Zerkleinerungsstufe radial hintereinander und auch wenigstens zwei derartige Zerkleinerungsstufen axial hintereinander angeordnet, so dass die durch den Homogenisierer geförderte Biomasse wenigstens durch zwei oder drei Zerkleinerungsstufen gefördert wird. Die Breite des Spalts oder die Anzahl der Scherkanten oder Schergegenkanten kann von Stufe zu Stufe variieren, vorzugsweise so, dass die Spaltbreite sich von Stufe zu Stufe in Förderrichtung verringert oder die Anzahl der Kanten sich in Förderrichtung von Stufe zu Stufe vergrößert, was auch den Fall einer Kombination beider Variationsmöglichkeiten einschließt. Die Verringerung der Spaltbreite oder die Vergrößerung der Anzahl der Kanten muss auch nicht bei jeder der Zerkleinerungsstufen auf die jeweils nächste vorgenommen werden, grundsätzlich genügt es, wenn eine Verringerung der Spaltbreite oder eine Vergrößerung der Anzahl der Scher- oder Schergegenkanten nur einmal zwischen zwei aufeinander folgenden Stufen stattfindet. Eine Variation der genannten Art zwischen mehr als nur zwei der Zerkleinerungsstufen wird jedoch bevorzugt.

Ist sowohl der Zerkleinerer als auch der Homogenisierer vorgesehen, ist es vorteilhaft, wenn der Homogenisierer im Gesamtstrom oder einem Teilstrom der Biomasse stromabwärts von dem Zerkleinerer angeordnet ist. Die beiden Komponenten sind vorzugsweise derart aufeinander abgestimmt, dass der Zerkleinerer eine Zerkleinerung der Feststoffbestandteile auf eine maximale Erstreckung sicherstellt, die nicht größer ist als ein größter lichter Abstand, den in Umfangsrichtung nächstbenachbarte Schermesser oder in radialer Richtung die Scherkanten radial nächstbenachbarter Scherkränze des Homogenisierers voneinander haben. Der Abstand in Umfangsrichtung wird an einem Scherkranz und der Abstand in radialer Richtung zwischen den zwei Scherkränzen der in Strömungsrichtung ersten Scherstufe, d. h. der Eingangsstufe des Homogenisierers gemessen.

Falls die Biomasse am Auslass des Zerkleinerers nur noch Feststoffpartikel mit einer maximalen Größe aufweist, die eine weitgehende Angleichung der genannten Kennlinien m(t) der unterschiedlichen Inhaltsstoffe dieser Biomasse gewährleistet, kann auf den Homogenisierer verzichtet werden. Diese größte Erstreckung der Partikel sollte 4 mm, besser 2 mm und noch besser 1 mm, nicht überschreiten. Falls der Homogenisierer wie bevorzugt zusätzlich zu dem Zerkleinerer vorgesehen ist, kann die Partikelgröße in dem vom Zerkleinerer behandelten Strom der Biomasse durch Messung im Strom direkt oder auf der Basis beispielsweise der für den Zerkleinerer erforderlichen Antriebsleistung oder einer stromauf oder stromab von dem Zerkleinerer angeordneten Fördereinrichtung indirekt ermittelt werden, um in Abhängigkeit von der ermittelten maximalen Erstreckung der Partikel die im Zerkleinerer behandelte Biomasse wahlweise entweder durch den Homogenisierer zu führen oder an diesem vorbei zum Auslass zu leiten.

Der Behälter ist in bevorzugten Ausführungen entweder ein Vorlagebehälter für Biomasse, die zumindest im Wesentlichen aus festen Bestandteilen besteht, wie beispielsweise Silage, Getreide, Rüben, Kartoffeln oder anderen landwirtschaftlichen Produkten, oder ein Anmischbehälter zum Mischen einer flüssigen Biomasse mit derartigen Festbestandteilen. Als Vorlagebehälter sollte der Behälter so groß sein, dass eine Füllung wenigstens dem Tagesbedarf des Fermenters entspricht. Falls solch ein Vorlagebehälter Bestandteil der erfindungsgemäßen Vorrichtung ist, kann der Einlass des Behälters den genannten Einlass der Vorrichtung bilden.

Ein Anmischbehälter ist vorteilhaft, falls die Biomasse nicht nur in eine Richtung von dem wenigstens einen Einlass zu dem wenigstens einen Auslass, sondern zwecks Einstellung ihrer Viskosität auch zurückgeführt oder in einem Kreis geführt werden soll. Dabei ist von Vorteil, wenn aus dem Anmischbehälter bereits mittels des Zerkleinerers zerkleinerte Biomasse vor den Anmischbehälter zurückgefördert werden kann, vorzugsweise auch vor den Zerkleinerer. Falls die Vorrichtung über den Homogenisierer verfügt, ist es alternativ oder vorzugsweise ergänzend zu dieser Rückführung vorteilhaft, wenn mittels des Homogenisierers behandelte Biomasse in den Anmischbehälter eingeleitet werden kann und hierfür eine Rückführleitung und ein Steuermittel für eine wahlweise Weiterleitung oder Rückführung vorgesehen sind.

In einer Weiterentwicklung ist der Homogenisierer in einer Leitung angeordnet, die vor dem Anmischbehälter abzweigt, so dass mittels des Zerkleinerers zerkleinerte Biomasse wahlweise in den Anmischbehälter oder zu dem Homogenisierer gefördert werden kann. Im Falle der bevorzugten Rückführbarkeit von Biomasse aus dem Anmischbehälter ist die Abzweigung für den Homogenisierer vorzugsweise stromab von einer Stelle im Leitungssystem angeordnet, bei welcher die Biomasse aus dem Anmischbehälter zurück in die in den Anmischbehälter führende Leitung gegeben wird.

Was die Rückführung von Biomasse aus dem Anmischbehälter betrifft, so ist vorteilhaft, wenn diese zurückgeführte Biomasse wahlweise stromauf oder stromab von dem Zerkleinerer wieder in die Leitung eingeleitet werden kann. Die Auswahl des Orts, zu dem die Biomasse aus dem Anmischbehälter zurückgeführt wird, erfolgt vorzugsweise automatisch anhand einer durch Messung direkt oder auf der Basis einer Förderleisung indirekt ermittelten Viskosität. Hierfür kann die Viskosität in dem Anmischbehälter oder in einer aus dem Anmischbehälter führenden Rückführleitung mittels eines Viskosimeters gemessen werden. Bevorzugt, da preiswerter, wird die Viskosität indirekt auf der Basis der für eine Fördereinrichtung erforderlichen Antriebsleistung ermittelt.

In einer Weiterentwicklung umfasst die Vorrichtung auf der Plattform auch eine Einrichtung zur Behandlung von Biomasse, die tierische Fette enthält. Die Einrichtung arbeitet vorzugsweise separat von den bereits erläuterten anderen Komponenten der Vorrichtung, könnte grundsätzlich aber auch in einem Kreislauf für die Behandlung der weniger kritischen Biomasse integriert sein, falls ein solcher Kreislauf vorhanden ist. Die Einrichtung für die Behandlung der tierischen Fette enthaltenden Biomasse umfasst einen Zerkleinerer für diese Fraktion. Der Zerkleinerer ist vorzugsweise ein Ultraschall-Zerkleinerer. Vorteilhafte Ultraschall-Zerkleinerer werden in der DE 10 2005 030 895 A1 offenbart. Die Einrichtung zur Behandlung der tierische Fette enthaltenden Biomasse umfasst in vorteilhaften Ausführungen ferner einen Sammelbehälter für diese Biomasse und eine eigene Förder- und Dosiereinrichtung, mittels der die Biomasse aus dem Sammelbehälter in und durch den Zerkleinerer förderbar ist. Diese Fraktion wird vorzugsweise mit der weiteren, auf der Plattform behandelten Biomasse gemischt, vorzugsweise auch noch auf der Plattform stromauf von dem wenigstens einen Auslass.

Vorteilhafterweise sind die Komponenten der Vorrichtung so miteinander vernetzt, dass die eingesetzte Biomasse zu einer Suspension mit in Flüssigkeit gleichmäßig verteilten Partikeln einer maximalen Größe von höchstens wenigen Millimetern zerkleinert, gemischt und dispergiert und somit die Ertragsausbeute an Biogas erhöht wird. Die für den Vergasungsvorgang im Fermenter erforderliche Verweildauer kann wesentlich verringert und somit die Wirtschaftlichkeit der Biogasanlage gegenüber herkömmlichen Systemen erhöht werden.

Weitere vorteilhafte Merkmale der Erfindung werden auch in den Unteransprüchen und deren Kombinationen beschrieben.

Nachfolgend wird ein Ausführungsbeispiel der Erfindung anhand von Figuren erläutert. An dem Ausführungsbeispiel offenbar werdende Merkmale bilden je einzeln und in jeder Kombination die Gegenstände der Ansprüche und die vorstehend beschriebenen Ausgestaltungen weiter. Es zeigen:
- Fig. 1: eine erfindungsgemäße Vorrichtung,
- Fig. 2: eine Container-Plattform und darin aufgenommene Komponenten der Vorrichtung,
- Fig. 3: einen Homogenisierer,
- Fig. 4: ein Detail eines Scherwerks des Homogenisierers,
- Fig. 5-7: jeweils einen Ultraschall-Zerkleinerer mit piezokeramischen Schwingelementen zur Aufnahme von unterschiedlichen Ansteuerungsfrequenzen,
- Fig. 8: einen Zerkleinerer und
- Fig. 9: ein Flussdiagramm einer Zerkleinerung und Dispergierung in mehreren Stufen.

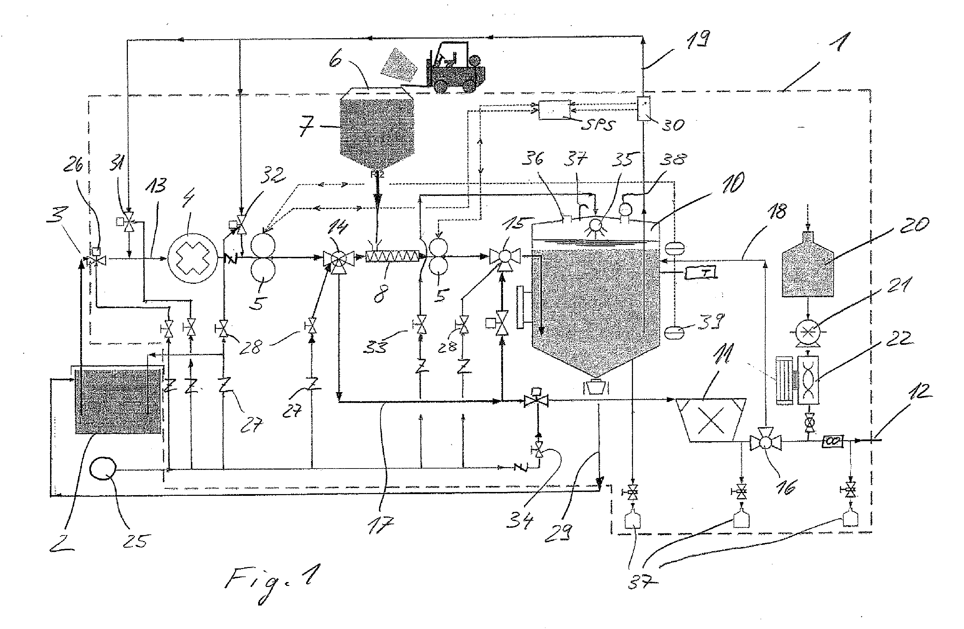

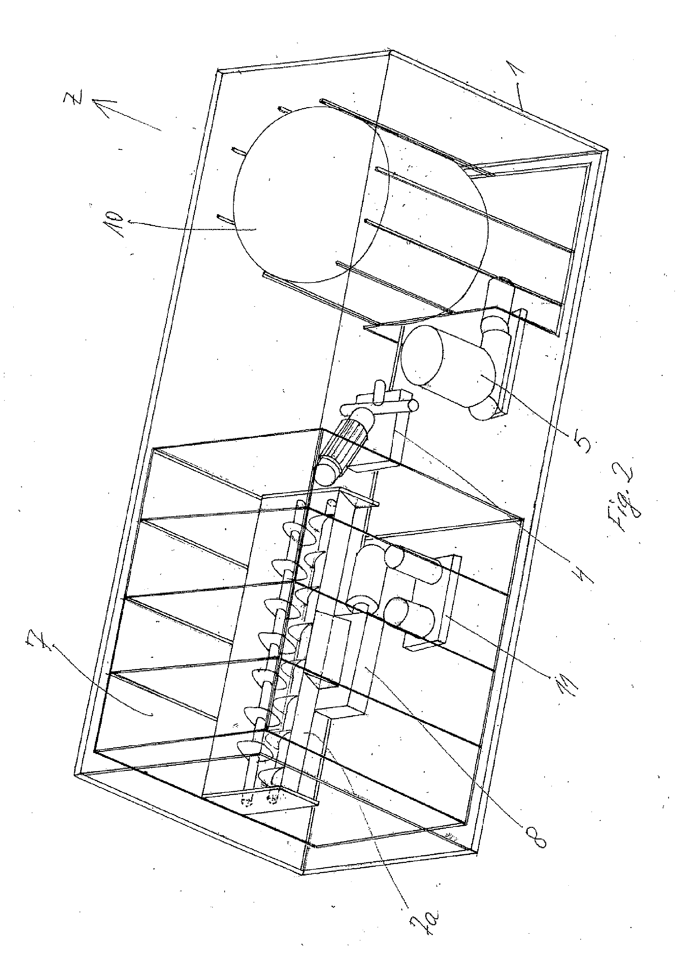

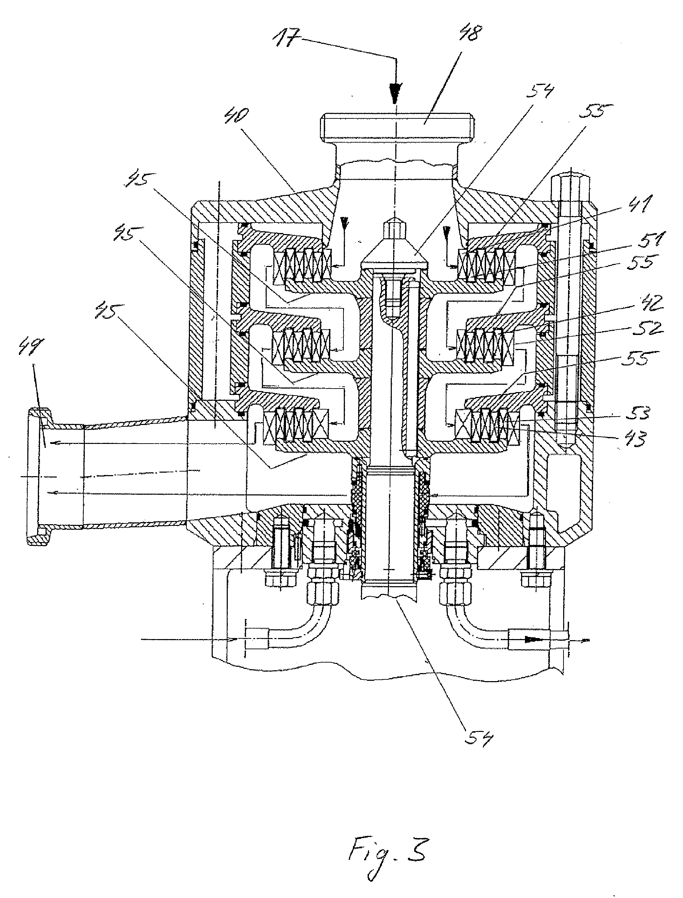

Figur 1 zeigt in einem Verfahrensfließbild eine erfindungsgemäße Vorrichtung mit auf einer gemeinsamen Plattform 1 angeordneten Komponenten für die Zerkleinerung und Homogenisierung von Biomasse. Die Plattform 1 ist in einer gestrichelten Linie dargestellt. Sie bildet einen Container und wird im Folgenden als "Container-Plattform" bezeichnet. Die gestrichelte Linie umgibt die Komponenten, die in der Container-Plattform 1 installiert sind. Die Vorrichtung dient der Aufbereitung der rohen Biomasse für einen Fermenter einer Biogasanlage.

In dem Container 1 sind unter anderem
- ein mechanischer Zerkleinerer 4,
- Pumpen 5,
- ein Vorlagebehälter 7,
- eine Mischeinrichtung 8,
- ein Anmischbehälter 10 und
- ein Homogenisierer 11
angeordnet.

Die Container-Plattform 1 weist Einlässe 3 und 6 für zu behandelnde Biomassen und einen Auslass 12 für die behandelte Biomasse auf. Sie ist über den Auslass 12 an den Fermenter der Biogasanlage angeschlossen oder anschließbar. Der Einlass 3 dient als Anschluss an eine am Ort einer Biogasanlage vorhandene Vorgrube 2. Durch den Einlass 3 kann fließfähige Biomasse, vorzugsweise Gülle, die mit einer oder mehreren anderen Flüssigfraktion(en) vermischt sein kann, beispielsweise mit Gärrückständen, aus der Vorgrube 2 durch eine Hauptleitung 13 in den Zerkleinerer 4 geführt werden. Diese Fraktion ist über den Zerkleinerer 4 der Mischeinrichtung 8 zuführbar. Über den Einlass 6 wird der Vorlagebehälter 7, ein Vorlagesilo, mit Biomasse befüllt, die einen großen Feststoffanteil enthält, beispielsweise Silage. Diese Fraktion wird im Vorlagebehälter 7 gelagert und gelangt über eine Austragseinrichtung mit zwei Schnecken in die Mischeinrichtung 8. Die Schnecken der Austragseinrichtung sind in einem konischen unteren Teil des Vorlagebehälters 7 liegend angeordnet. Aus dem Vorlagebehälter 7 können zusätzlich zu der Fraktion aus der nicht in der Container-Plattform 1 angeordneten Vorgrube 2 weitere Biomassen unterschiedlichster Art zu einer Vorzerkleinerung in die Mischeinrichtung 8 gefüllt werden.

Der Zerkleinerer 4 und die Mischeinrichtung 8 können Komponenten sein, die in Biogasanlagen bereits ihre Tauglichkeit bewiesen haben. Die Mischeinrichtung 8 kann so ausgebildet sein, dass sie über das Mischen hinaus auch fördert oder zerkleinert.

Der Vorlagebehälter 7 ist von oben befüllbar, beispielsweise wie dargestellt mittels eines Gabelstaplers oder eines anderen vor Ort verfügbaren Transportmittels. Der nach oben offene Einlass 6 des Vorlagebehälters 7 kann mittels eines Verschlusselements, beispielsweise einer Klappe oder eines Deckels, verschließbar sein. Das Verschlusselement kann insbesondere ein Wandungsteil des Containers 1 sein. Unter der Austragseinrichtung ist ein Auslass des Vorlagebehälters 7 geformt, zweckmäßigerweise einfach als Öffnung. Der Auslass ist lotrecht über einem Einlass der Mischeinrichtung 8 angeordnet, so dass die trockene Biomasse mittels der Austragseinrichtung durch den Auslass des Vorlagebehälters 7 und dann einfach mittels Schwerkraft der Mischeinrichtung 8 zuführbar ist.

Die Hauptleitung 13 führt die Biomasse von dem Einlass 3 über den Zerkleinerer 4 und die Mischeinrichtung 8 in den Anmischbehälter 10. Die Hauptleitung 13 ist mittels eines Steuermittels 26, im Ausführungsbeispiel ein Absperrventil, das zwischen dem Einlass 3 und dem Zerkleinerer 4 angeordnet ist, absperrbar. In der Hauptleitung 13 sind zwischen dem Zerkleinerer 4 und der Mischeinrichtung 8 eine Pumpe 5 und ein Steuermittel 14 angeordnet. Zwischen der Mischeinrichtung 8 und dem Anmischbehälter 10 sind in der Hauptleitung 13 eine weitere Pumpe 5 und ein weiteres Steuermittel 15 angeordnet. Die Steuermittel 14 und 15 sind Mehrwegeventile, im Ausführungsbeispiel Dreiwegeventile.

Die Mischeinrichtung 8 vermischt die flüssige oder zumindest fließfähige Biomasse in der Hauptleitung 13 mit der im Wesentlichen aus festen Bestandteilen bestehenden Biomasse aus dem Vorlagebehälter 7. Die Mischeinrichtung 8 ist auch gleichzeitig eine Fördereinrichtung für die Förderung der Biomasse in Richtung auf den Anmischbehälter 10. Die Pumpen 5 sind vorzugsweise Drehkolbenpumpen.

In dem Anmischbehälter 10 werden die unterschiedlichen Fraktionen auf eine vorgegebene Viskosität eingestellt. Im Behälter 10 kann ein Rührwerk angeordnet sein. Für die Einstellung der Viskosität kann Mischung aus dem Behälter 10 abgezogen und in den Behälter 10 zurückgeführt werden. Falls erforderlich wird die Mischung vor dem Wiedereinleiten über den Zerkleinerer 4 oder den Homogenisierer 11 zurück geführt, um die Feststoffanteile nochmals einer Zerkleinerung zu unterziehen oder den zurück geführten Strom oder einen daraus gebildeten Teilstrom der Flüssig-Fest-Mischung in Form einer feinen Dispersion wieder in den Behälter 10 einzuleiten. Desweiteren besteht die Möglichkeit, zur Verringerung der Viskosität über ein Steuermittel 28 Wasser in den Behälter 10 einzuleiten.

Bei dem Steuermittel 14 zweigt von der Hauptleitung 13 eine Leitung 17 zu dem Homogenisierer 11 ab. Im Homogenisierer 11 wird diese Biomasse fein zerkleinert, dispergiert und in diesem Sinne homogenisiert. Im Homogenisierer 11 werden die Bestandteile der Biomasse soweit zerkleinert und vermischt, dass eine homogene Flüssigkeit von sahneförmiger Konsistenz entsteht, die als "Maissahne" charakterisiert werden kann. Die Feinzerkleinerung trägt dazu bei, dass die Biomasse im Fermenter rascher und intensiver mikrobiell abgebaut werden kann. Stromab von dem Homogenisierer 11 ist ein weiteres Steuermittel 16, im Ausführungsbeispiel ein weiteres Mehrwegeventil, angeordnet. Die im Homogenisierer 11 behandelte Biomasse kann über das Steuermittel 16 wahlweise entweder durch eine Rückführleitung 18 in den Anmischbehälter 10 oder in einem weiterführenden Teil der Leitung 17 zu dem Auslass 12 der Vorrichtung geleitet werden. Ferner ist die Aufteilung in zwei Teilströme möglich.

Die Steuermittel 14, 15 und 16 sind so ausgebildet, dass sie den ankommenden Strom unabhängig voneinander jeweils in einem Verhältnis, das von einer Steuerung automatisch vorgegeben und zur Erzeugung einer am Auslass 12 optimalen Biomasse angepasst wird, in Teilströme aufteilen können. In einer Vereinfachung kann aber auch eines oder können mehrere dieser Steuermittel so ausgebildet sein, dass stets nur ein Ausgang des jeweiligen Steuermittels offen und Durchfluss nur durch diesen Auslass möglich ist.

Bei dem Homogenisierer 11 handelt es sich um ein aus der Kosmetikindustrie bekanntes Dispergiergerät mit einem Elektromotor und einem feinen Schneid- oder Scherwerk, wie es zum Anrühren von Pasten und Salben verwendet wird.

Zwecks Umwälzung wird die im Anmischbehälter 10 befindliche Mischung aus Flüssigkeit und Festbestandteilen über eine Rückführleitung 19 in den Prozess zurückgeführt. Die Rückführleitung 19 umfasst eine aus dem Anmischbehälter 10 führende Steigleitung. Die Rückführleitung 19 verzweigt sich in zwei Leitungszweige, die stromauf von der Mischeinrichtung 8 in die Hauptleitung 13 führen. Der eine Leitungszweig führt stromauf und der andere stromab von dem Zerkleinerer 4 in die Hauptleitung 13. Falls die festen Bestandteile der Mischung aus dem Anmischbehälter 10 noch nicht ausreichend fein zerkleinert sind, wird die Mischung über ein absperrbares Steuermittel 31, im Ausführungsbeispiel ein Absperrventil, nochmals durch den Zerkleinerer 4 geführt. Reicht der Zerkleinerungsgrad aus, wird die Mischung unter Umgehung des Zerkleinerers 4 über ein absperrbares Steuermittel 32, im Ausführungsbeispiel ebenfalls ein Absperrventil, zu der Mischeinrichtung 8 geführt, um elektrische Antriebsenergie für den Zerkleinerer 4 einzusparen.

Falls die Biomasse stromab von dem Zerkleinerer 4 bereits fein genug ist, wird sie über das stromauf von der Mischeinrichtung 8 angeordnete Steuermittel 14 zum Homogenisierer 11 geführt, gegebenenfalls auch nur ein Teilstrom dieser Biomasse, und dort fein dispergiert. Da der Massenstrom der Hauptleitung 13 für den Homogenisierer 11 im Allgemeinen zu groß ist, kann der Strom der Leitung 17 vor dem Homogenisierer 11 geteilt und ein Teilstrom, beispielsweise die Hälfte, zum Homogenisierer 11 und der andere Teilstrom über das Steuermittel 15 in den Anmischbehälter 10 geführt werden.

Über die Einlässe 3 und 6 für die unterschiedlichen Fraktionen der Biomasse hinaus verfügt die Container-Plattform 1 auch über einen Wasseranschluss 25. Durch den Wasseranschluss 25 kann Wasser zum Verdünnen in den Strom der Biomasse oder zum Spülen von verschmutzten Komponenten eingespeist werden. Falls Komponenten, insbesondere Steuermittel, der Vorrichtung verstopft sein sollten, wird das Steuermittel 26 zwischen der Vorgrube 2 und dem Zerkleinerer 4 geschlossen und statt der Flüssigkeit aus der Vorgrube 2 die Spülflüssigkeit durch die Anlage, genauer gesagt durch das oder die verstopften Komponenten gepumpt. Hierfür sind die betreffenden Steuermittel über Steuerungsventile 28 und vorzugsweise Rückschlagventile 27 einzeln durchspülbar. Zum Verdünnen kann Wasser über ein Steuermittel 33 und ein vorgeschaltetes Rückschlagventil in den Anmischbehälter 10 gefördert werden. Das Wasser wird über Düsen 35 eingespeist, die in dem Anmischbehälter 10 angeordnet sind und in Doppelfunktion als Reinigungsdüsen dienen, um den Anmischbehälter 10 auch automatisch reinigen zu können. Wasser kann ferner über ein Steuermittel 34 und ein vorgeschaltetes Rückschlagventil zum Homogenisierer 11 geleitet werden. Die Steuermittel 33 und 34 sind beispielsweise einfache Absperrventile.

Die Container-Plattform 1 weist auch einen weiteren Auslass-Anschluss auf, über den ein Sumpf des Anmischbehälters 10 entleert werden kann. Der Anmischbehälter 10 ist über eine Leitung 29 und den weiteren Auslass-Anschluss für die Sumpfentleerung an die Vorgrube 2 angeschlossen oder an diese anschließbar.

Die Vorrichtung umfasst eine Steuerung SPS, die über die Steuermittel als Stellglieder, insbesondere die Steuermittel 14 bis 16 und 31 bis 34, die Behandlung der Biomasse steuert (und regelt). Die Steuerung SPS sorgt dafür, dass die in dem Anmischbehälter 10 enthaltene Mischung eine vorgegebene Viskosität zumindest nicht wesentlich überschreitet.

Ein Viskosimeter 30 misst die Viskosität der aus dem Anmischbehälter 10 zurückgeführten Mischung. Das Viskosimeter 30 ist außerhalb des Anmischbehälters 10 in der Rückführleitung 19 stromauf von der Verzweigung der Rückführleitung 19 angeordnet. Solange die Viskosität der Mischung größer ist als ein vorgegebener Viskositätswert, wird dem Anmischbehälter 10 über das Steuerungsmittel 33 Wasser zugeführt. Preiswerter und daher bevorzugter wird die Viskosität jedoch indirekt aus der Förderleistung einer oder mehrerer der Fördereinrichtungen der Vorrichtung ermittelt, beispielsweise der Pumpen 5 oder einer im Strom der Biomasse näher bei dem Fermenter angeordneten Fördereinrichtung, die vorzugsweise auf dem Plattform-Container 1 angeordnet ist. Am günstigsten ist es wenn die Leistung der letzten Fördereinrichtung vor dem Fermenter erfasst wird. Für die indirekte Ermittlung der Viskosität kann insbesondere die Änderung des Stromverbrauchs der betreffenden Fördereinrichtung erfasst werden. Die erfassten Abweichungen von einem Strommittelwert repräsentieren Abweichungen der Viskosität von einem gewünschten Mittelwert, auf den die am Ort der Erfassung vorliegende Mischung, bevorzugt die feine Dispersion am Auslass 12, eingestellt werden soll.

Um Biomasse, die in nennenswertem Umfang tierische Fette oder Rückstände von Antibiotika enthält, behandeln zu können, aber nicht mit den anderen Fraktionen mischen zu müssen, ist in der Container-Plattform 1 eine Einrichtung zur Behandlung der die tierischen Fette oder Antibiotika enthaltenden Biomasse separat von dem in und aus dem Anmischbehälter 10 führenden Leitungssystem angeordnet. Diese zusätzliche Einrichtung umfasst einen Sammelbehälter 20, in dem fetthaltige Abfälle, wie Schlacht- und Küchenabfälle, gesammelt werden. Die Container-Plattform 1 weist einen weiteren Einlass für diese Fraktion auf. Stromab von dem Sammelbehälter 20 ist ein Zerkleinerer 22 für die Zerkleinerung der aus dem Sammelbehälter 20 zugeleiteten Biomasse angeordnet. Der Zerkleinerer 22 ist ein Ultraschall-Zerkleinerer. Die Zufuhr zu dem Ultraschall-Zerkleinerer 22 erfolgt mittels einer Dosierpumpe 21, die in einer Leitung zwischen dem Sammelbehälter 20 und dem Ultraschall-Zerkleinerer 22 angeordnet ist. Die vom Ultraschall-Zerkleinerer 22 zerkleinerte Biomasse wird zwischen dem Steuermittel 16 und dem Auslass 12 in die Leitung 17 eingespeist.

Die Ausstattung der Container-Plattform 1 mit der zusätzlichen Einrichtung zur Aufbereitung der tierische Fette oder Antibiotika enthaltenden Biomasse ist in einer Grundvariante der Vorrichtung nicht enthalten. Die Einrichtung ist Bestandteil einer Ausbaustufe der Vorrichtung, findet vorzugsweise aber auch in der Container-Plattform 1 der Grundversion Platz, so dass die Grundversion problemlos mit dieser Einrichtung nachgerüstet werden kann.

In der Container-Plattform 1 kann eine Zugabeeinrichtung für Enzyme angeordnet sein, um der Biomasse an geeigneter Stelle, vorzugsweise zwischen dem Homogenisierer 11 und dem Auslass 12, in dosierter Menge Enzyme für eine Nachbehandlung beimischen zu können. Eine Beimischung im Behälter 10 ist ebenfalls denkbar, obgleich weniger bevorzugt.

Figur 2 zeigt die Container-Plattform 1 in einer perspektivischen Sicht. Die Plattform 1 weist einen Boden, eine Decke und Seitenwände auf. Die Plattform 1 ist durchsichtig dargestellt, so dass die aufgenommenen Komponenten und deren Anordnung zu erkennen sind. Die Container-Plattform 1 hat die Form eines länglichen Quaders. Der Quader ist zwischen 2 und 2,5 m breit, zwischen 2 und 4 m hoch und zwischen 5 und 10 m lang. Mit "Z" ist die vertikale Hochachse bezeichnet. Die Container-Plattform 1 besteht im Wesentlichen aus einem festen Gerippe von Streben, an dem die Außenwände und die Decke befestigt sind, so dass ein allseitig umschlossener Hohlraum erhalten wird, in dem die Komponenten der Vorrichtung angeordnet sind, die die Biomasse aufnehmen, führen, fördern und behandeln.

Der Vorlagebehälter 7 ist im Sinne einer möglichst effizienten Nutzung des verfügbaren Raums in das System aus Gerippe und Containerwänden integriert. indem ein Teil der Außenwände und der Decke gleichzeitig auch einen Teil der Wand und die Decke des Vorlagebehälters 7 bilden. Ferner erstrecken sich Streben des Gerippes schräg zur Hochachse Z geneigt nach innen. Diese Streben tragen einen Boden 7a des Vorlagebehälters 7, so dass sich der Vorlagebehälter 7 in Richtung auf seinen Auslass nach unten verjüngt. Die Wände des Vorlagebehälters 7 sind zumindest innen glatt und weisen einen niedrigen Reibungskoeffizienten auf, um eine Brückenbildung der aufgenommenen Biomasse zu verhindern. Die Wände können beispielsweise an ihrer Innenseite mit einem Gleitmaterial, vorzugsweise einem Kunststoff, beschichtet sein. Zusätzlich oder stattdessen kann eine Rütteleinrichtung vorgesehen sein, mit der die Biomasse im Vorlagebehälter 7 durchgerüttelt werden kann.

Der Anmischbehälter 10 ist kein integrierter, sondern ein eingebauter Behälter mit einer runden, um die Hochachse Z zylindrisch umlaufenden Seitenwand.

Die beiden Behälter 7 und 10 sind in Längsrichtung der Container-Plattform 1 gesehen je nahe bei den Stirnseiten der Container-Plattform 1 angeordnet, wobei eine der Stirnwände gleichzeitig auch eine Seitenwand des Vorlagebehälters 7 bildet. Die Anordnung der Behälter 7 und 10, die einen großen Teil des Volumens der Container-Plattform 1 beanspruchen, trägt zur effizienten Nutzung des verfügbaren Raums bei. Für den Zerkleinerer 4, die Mischeinrichtung 8, den Homogenisierer 11, die Pumpen 5 und das Leitungssystem mit den Steuermitteln verbleibt genug Raum zwischen den Behältern 7 und 10 und unter dem Vorlagebehälter 7. Der Zerkleinerer 4, die Mischeinrichtung 8, der Homogenisierer 11 und die Pumpen 5 sind leicht zugänglich und so angeordnet und montiert, dass sie einzeln ausgetauscht werden können, ohne den Aus- oder Umbau einer der jeweils anderen Komponenten zu erfordern. Dies erleichtert auch ein nachträgliches Um- oder Aufrüsten der Vorrichtung.

Die Container-Plattform 1 weist eine Tür auf, durch die man den Innenraum für Reparatur- und Wartungsarbeiten sowie Inspektionen und gegebenenfalls Einstellarbeiten betreten kann. Die Tür befindet sich vorzugsweise an einer Längsseite in einem Bereich zwischen den Behältern 7 und 10.

Fig. 3 zeigt den Homogenisierer 11 in einem Längsschnitt entlang einer zentralen Längsachse, die gleichzeitig eine Rotationsachse R eines Scherwerks des Homogenisierers 11 ist. Der Homogenisierer 11 weist ein Gehäuse 50 mit einem Einlass 58 (Einfüllstutzen) für die zu dispergierende Biomasse und einem Auslass 59 für die dispergierte Biomasse auf. Das Scherwerk besteht aus einem in dem Gehäuse 50 ortsfesten Stator und einem Rotor 40, der in dem Gehäuse 50 um die Rotationsachse R drehbar gelagert ist. Der Stator umfasst eine erste Gruppe von Scherkränzen 51, eine zweite Gruppe von Scherkränzen 52 und eine dritte Gruppe von Scherkränzen 53. Die Scherkränze 51 bis 53 sind unbeweglich mit dem Gehäuse 50 verbunden. Die zur gleichen Gruppe gehörenden Scherkränze, d. h. die Scherkränze 51 der ersten Gruppe, die Scherkränze 52 der zweiten Gruppe und die Scherkränze 53 der dritten Gruppe, umgeben einander je in einem radialen Abstand konzentrisch, während die Scherkränze 51 bis 53 von Gruppe zu Gruppe axial voneinander beabstandet sind. Der Rotor 40 weist eine erste Gruppe von Scherkränzen 41, eine zweite Gruppe von Scherkränzen 42 und eine dritte Gruppe von Scherkränzen 43 auf. Die Scherkränze 41 bis 43 sind mit einer um die Rotationsachse (R) drehbaren Zentralwelle 44 drehsteif verbunden. Die Scherkränze 41 sind auf einem ersten Träger 45, die Scherkränze 42 auf einem zweiten Träger 45 und die Scherkränze 43 auf einem dritten Träger 45 angeordnet. Die Träger 45 ragen von der Welle 44 nach außen ab und sind axial voneinander beabstandet. Sie bilden je einen Boden für die zu behandelnde Biomasse. Die Scherkränze 41 und 51 der beiden ersten Gruppen umgeben einander konzentrisch, wobei in radialer Richtung auf einen der Scherkränze 41 je einer der Scherkränze 51 folgt, so dass in radialer Richtung eine alternierende Abfolge von Rotor-Scherkränzen 41 und Stator-Scherkränzen 51 erhalten wird. Zwischen den radial benachbarten Scherkränzen 41 und 51 verbleibt jeweils um die Rotationsachse R umlaufend ein schmaler Spalt. Bei den Scherkränzen 42 und 52 der beiden zweiten Gruppen und den Scherkränzen 43 und 53 der beiden dritten Gruppen herrschen die gleichen Verhältnisse.

Jeder der Scherkränze 41 bis 43 weist mehrere Schermesser auf, die je eine Scherkante bilden. Die Scherkränze 51 bis 53 weisen ebenfalls je mehrere Schermesser auf, von denen jedes eine Schergegenkante für die Scherkanten des radial innen oder außen nächsten der Scherkränze 41 bis 43 bildet. Die Scherkanten und Schergegenkanten sind bei jedem der Scherkränze 41 bis 43 und 51 bis 53 um die Rotationsachse R gleichmäßig verteilt angeordnet. Die Scherkanten und Schergegenkanten sind zu der Rotationsachse R parallel. Sie könnten zur Rotationsachse R eine Neigung aufweisen, sollten aber jedenfalls eine zur Rotationsachse R parallele Richtungskomponente haben. Die Scherkanten und Schergegenkanten sind gerade, könnten aber auch einen gekrümmten Verlauf aufweisen.

Fig. 4 zeigt einen Bogenabschnitt eines der Scherkränze 41 und einen Bogenabschnitt eines radial nächsten Scherkranzes 51. Der Scherkranz 41 besteht aus Schermessern 46, die von einem Träger 45 des Rotors 40 axial abragen. Der Scherkranz 51 besteht aus Schermessern 56, die von einem der Träger 55 des Gehäuses 50 axial abragen und die Schermesser 46 axial überlappen. Die Träger 45 und 55 sind axial zueinander versetzt. Die Schermesser 46 und 56 ragen von ihrem jeweiligen Träger 45 oder 55 in Richtung auf den jeweils anderen Träger 55 oder 45 zu. An der in Bezug auf die Drehrichtung des Rotors 40 vorlaufenden Seite ist an jedem der Schermesser 46 eine der genannten Scherkanten 47 und an jedem der Schermesser 56 eine der genannten Schergegenkanten 57 geformt. Zwischen den in Umfangsrichtung um die Rotationsachse R benachbarten Schermessern 46 verbleibt jeweils ein lichter Abstand, ebenso zwischen den Schermessern 56, so dass die Biomasse radial durch einen der Scherkränze 41 und 51 in den zwischen nächstbenachbarten Scherkränzen 41 und 51 verbleibenden schmalen Spalt und aus diesem durch den anderen der Scherkränze 41 und 51 strömen kann. Die weiteren Scherkränze 41 bis 43 und 51 bis 53 sind in gleicher Weise geformt und innerhalb der jeweils einander zugeordneten ersten, zweiten und dritten Gruppen angeordnet, so dass innerhalb der einander zugeordneten Gruppen jeweils zwei radial nächste Scherkränze 41 und 51, 42 und 52 sowie 43 und 53 zusammenwirkend eine Zerkleinerungsstufe bilden.

Um die Biomasse zu dispergieren, wird der Rotor 50 durch einen Motor mit großer Geschwindigkeit in Rotation versetzt, vorzugsweise mit einer Umfangsgeschwindigkeit, die am radial äußersten Scherkranz jeder Axialstufe wenigstens 30 m/s beträgt. Zur Kühlung des Scherwerks wird Kühlflüssigkeit durch das System geführt. Die Biomasse, optional mit Wasser angereichert, wird am Einlass 58 zugeführt. Sie wird im Gehäuse 50 so geführt, dass sie die Gruppen von Scherkränzen 41 und 51, 42 und 52 sowie 43 und 53 jeweils von radial innen nach außen durchströmt. Ferner ist der Rotor 40 so geformt und angeordnet, dass er der Biomasse einen Drehimpuls um die Rotationsachse R verleiht und die erzeugte Fliehkraft die Förderung der Biomasse durch den Homogenisierer 11 unterstützt. Gegebenenfalls reicht der Drehantrieb des Rotors 40 für die Förderung der Biomasse im Homogenisierer 11 aus, vorteilhafterweise unterstützt die Fördereinrichtung 5 die Förderung durch den Homogenisierer 11. Die Biomasse wird nacheinander durch sämtliche Zerkleinerungsstufen aller Gruppen von Scherkränzen 41 bis 43 und 51 bis 53 gefördert. Der Strömungspfad durch das Gehäuse 50 ist in Figur 3 eingezeichnet. Beim Durchlauf der Biomasse durch die Kammern des Scherwerks erfolgt eine sukzessiv feinere Zerkleinerung der festen Bestandteile bis auf eine Partikelgröße von Mikro- und Nanometern. Die Partikel weisen eine größte Erstreckung von vorteilhafterweise höchstens 2 mm, bevorzugter höchstens 1 mm auf.

Die Drehgeschwindigkeit des Rotors 40 wird vorzugsweise so hoch und die radialen Abstände zwischen den miteinander scherenden Schermessern 46 und 56 werden vorzugsweise so klein gewählt, dass in der Flüssigkeit der Biomasse Kavitationsbläschen auftreten, die zu einer feinen Dispergierung der Biomasse beitragen, indem die Kavitationskräfte die festen Bestandteile zerreißen.

Fig. 5 zeigt den Ultraschall-Zerkleinerer 22, im Ausführungsbeispiel ein Ultraschall-Rohrschwinger, in einem Längsschnitt und drei Querschnitten. Der Ultraschall-Zerkleinerer 22 umfasst ein Rohr 23 und in oder an der Rohrwandung angeordnete Ultraschallerzeuger 24a, 24b und 24c auf. Die Schallerzeuger 24a bilden eine erste Gruppe, die Schallerzeuger 24b eine zweite und die Schallerzeuger 24c eine dritte Gruppe usw. Die Schallerzeuger der jeweils gleichen Gruppe sind in Längsrichtung des Rohrs 23 auf der gleichen Höhe angeordnet, während die Schallerzeuger von Gruppe zu Gruppe in Längsrichtung des Rohrs 23 zueinander versetzt sind. Die Schallerzeuger 24a, 24b und 24c sind innerhalb der jeweiligen Gruppe in einem Winkel α von 72° um ein Zentrum A des jeweiligen Rohrquerschnitts in oder an der Rohrwandung zueinander versetzt positioniert. Da der gesamte Rohrschwinger mit dreißig Schallerzeugern bestückt ist und jede Ebene davon fünf Schallerzeuger aufnimmt, existieren somit sechs Ebenen mit jeweils fünf Schallerzeugern. Die erste Ebene mit den fünf Schwingelementen 24a weist nur einmal ein Schwingelement mit schraffierter Zeichnung aus. Die anderen vier Schwingelemente sind aber auf die gleiche Ansteuerungs-Frequenz abgestimmt. Das gilt für alle nachfolgenden Ebenen, d. h. gleiche Schraffur gleiche Ansteuerungs-Frequenz. Die erste Ebene wird mit einer Frequenz von 20 kHz, die zweite mit einer Frequenz von 100 kHz, und die dritte Ebene wird mit 40 kHz angesteuert. Die nächsten Ebenen - vier, fünf und sechs - sind eine Wiederholung der ersten drei Ebenen. Die sechste Ebene ist nur gestrichelt dargestellt. Die Schwingelemente 24a-c sind von Gruppe zu Gruppe axial fluchtend in oder an der Rohrwandung positioniert. Durch eine Frequenzansteuerung mittels eines Zufallgenerators der Schwingelemente kann sich für einzelne Partikel der Biomasse keine Fluchtströmung aufbauen, die ein Ausweichen der Partikel zwischen den implodierenden Kavitationsbläschen ermöglichen würde. Da bei der hier vorgeschlagenen Technik keine stehenden Wellen entstehen können, ist ein maximales Zerreißen der Partikel bis in den Nanobereich gegeben.

Fig. 6 zeigt eine modifizierte Anordnung von Schallerzeugern 24a-c. Die Schallerzeuger 24b sind hier um α/2, also um 36°, der Drehwinkelposition nach gegenüber den axial links und rechts davon beabstandet angeordneten Schallerzeugern 24a und 24c versetzt. Hier gilt ebenfalls die kontinuierliche Wiederholung der Anordnung der Schallelemente bis zur Ebene sechs.

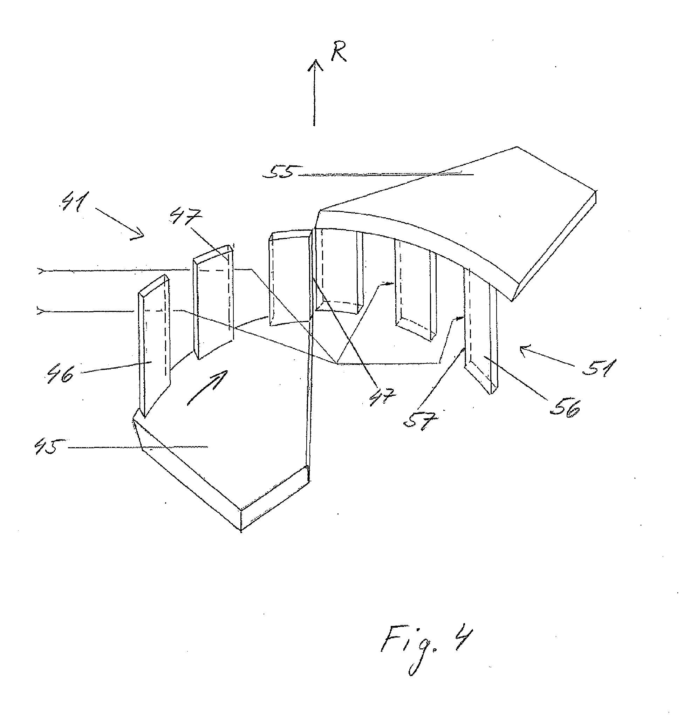

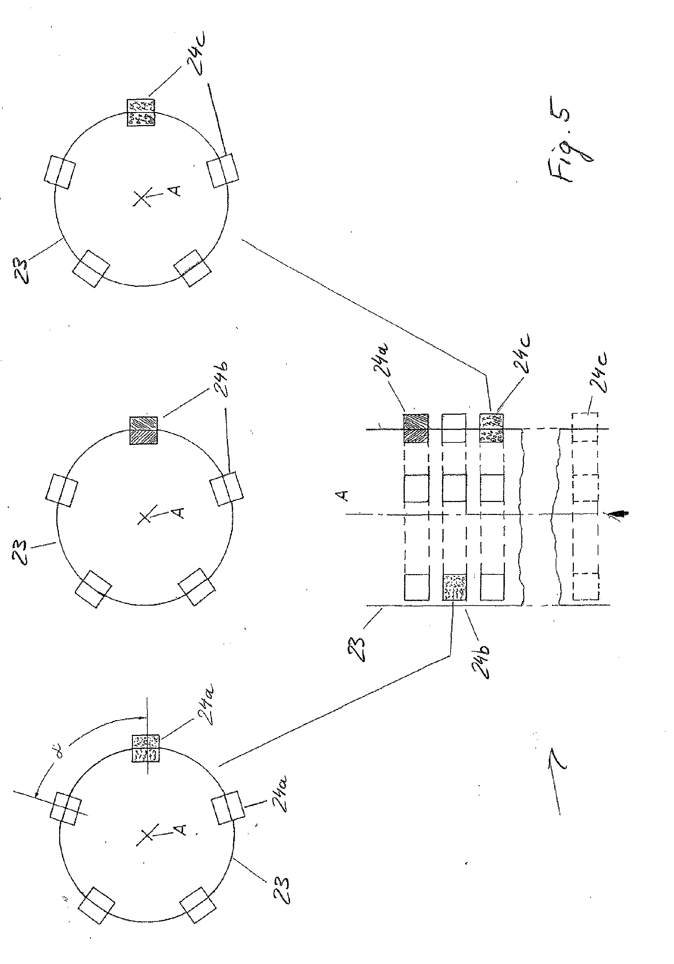

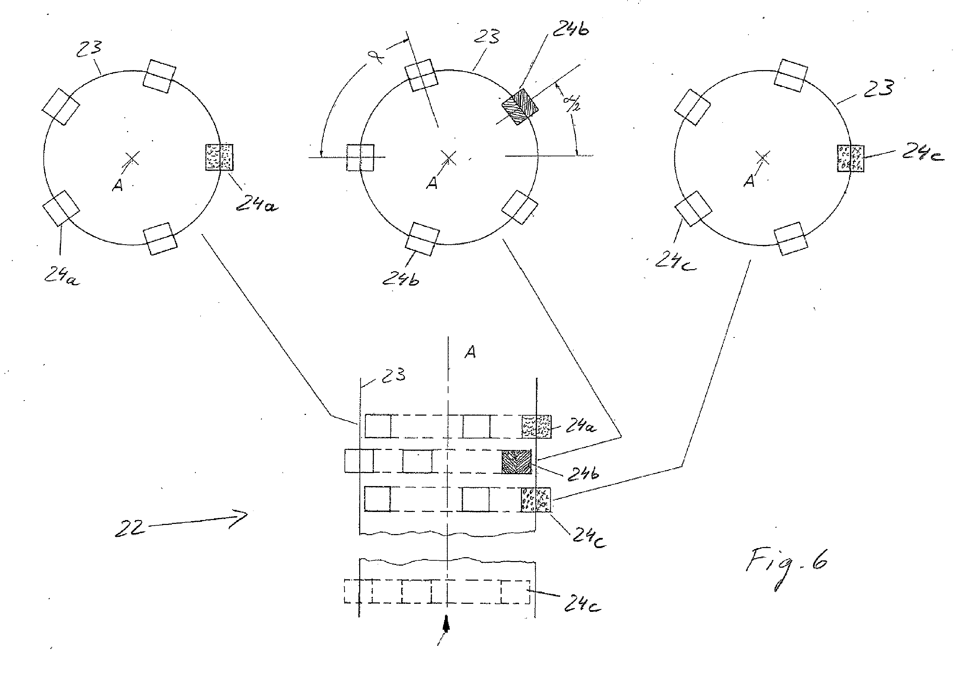

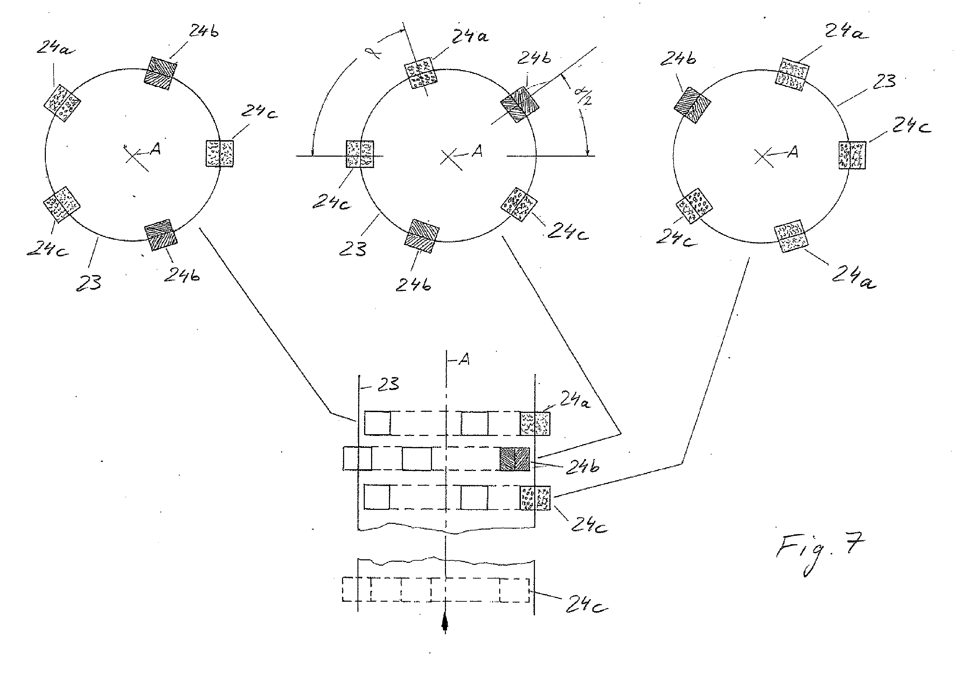

Fig. 7 zeigt noch eine andere Anordnung von Schallerzeugern 24a-c. Die drei der Ansteuerungsfrequenz nach unterschiedlichen Schwingelemente 24a, 24b und 24c sind auf jeder der Ebenen positioniert, wobei sich zwei der drei Schwingelemente auf einer Ebene wiederholen. In Umfangsrichtung um das Zentrum A ergibt sich für die erste Ebene die Abfolge 24a-24b-24c-24b-24c. Die zweite Ebene ist wie bei der Anordnung der Figur 6 um α/2 = 36° verdreht, so dass die Schallerzeuger jeder Ebene zu den Schallerzeugern der axial benachbarten Ebene in Umfangsrichtung jeweils "auf Lücke" positioniert sind. Zusätzlich kann noch die Abfolge verändert werden, wie in dem rechten Querschnitt der Figur 7 angedeutet ist. Diese Veränderung der jeweiligen Position auf einer Ebene setzt sich fort bis zur Ebene sechs.

Die Variationen der Anordnungen der Schallelemente sollen ein Maximum an Kavitationsbläschen erzeugen.

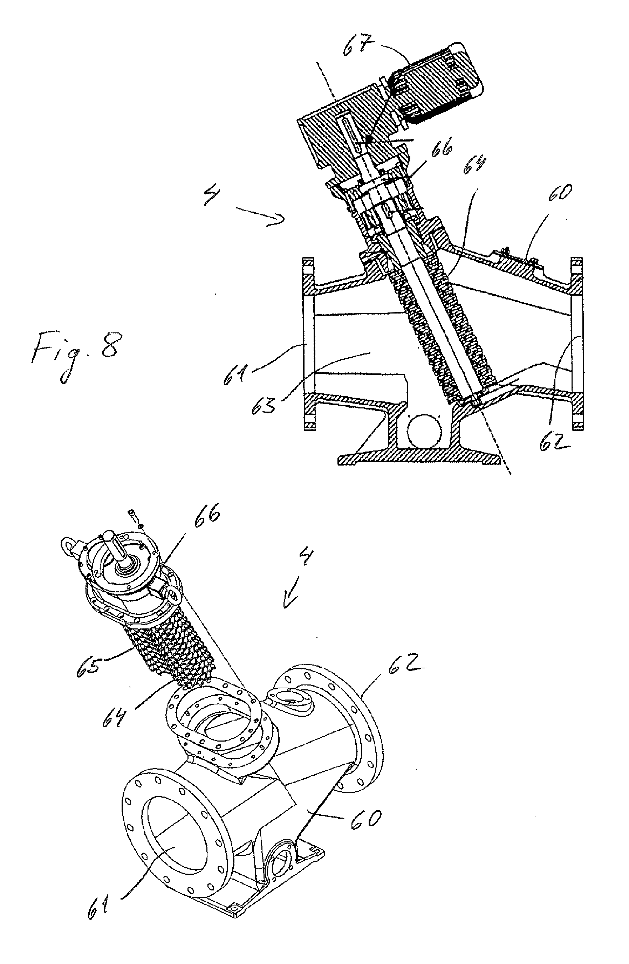

Figur 8 zeigt den Zerkleinerer 4 in einem Schnitt und einer Ansicht. Der Zerkleinerer 4 weist ein Gehäuse 60 mit einem Einlass 61 und einem Auslass 62 auf, zwischen denen das Gehäuse 60 einen Strömungabschnitt 63 der Hauptleitung 13 bildet. Im Strömungsabschnitt 63 sind zwei Messerwellen 64 und 65 quer zu der Strömungsrichtung der Biomasse außenachsig nebeneinander angeordnet. In der Ansicht ist das ausziehbare Schneidwerk mit den Messerwellen 64 und 65 und einem Getriebe 66 für deren Drehantrieb bereit für die Montage im Gehäuse 60 dargestellt. Jede der Messerwellen 64 und 65 weist axial nebeneinander eine Vielzahl von Schneidmessern mit vorstehenden Schneidzähnen auf. Zwischen nächstbenachbarten Schneidmessern verbleibt jeweils ein axialer Abstand. Die Messerwellen 64 und 65 greifen ineinander, d. h. in die Lücken zwischen den Schneidmessern der einen Messerwelle greifen die Schneidmesser der jeweils anderen ein. Die Messerwellen 64 und 65 werden von einem Motor 67 über das Getriebe 66 gegenläufig drehangetrieben. Zwischen den Messerwellen 64 und 65 verbleibt ein Spalt, durch den die Biomasse gefördert und im kämmenden Eingriff der Messerwellen 64 und 65 zerkleinert wird. Die Feststoffbestandteile der Biomasse werden beim Durchgang durch den Spalt zerschnitten, zerrissen und zerdrückt. Die Weite des Spalts, d. h. der Abstand zwischen den Achsen der Messerwellen 64 und 65, ist vorzugsweise so gewählt, dass die Feststoffbestandteile nach dem Durchgang durch den Spalt eine größte Erstreckung von höchstens 50 mm haben. Noch bevorzugter ist die Spaltweite so gewählt, dass ihre größte Erstreckung höchstens 30 mm beträgt. Für die Zerkleinerung ist es günstig, wenn die Messerwellen 64 und 65 mit unterschiedlicher Umfangsgeschwindigkeit angetrieben werden, zueinander also eine Geschwindigkeitsdifferenz aufweisen.

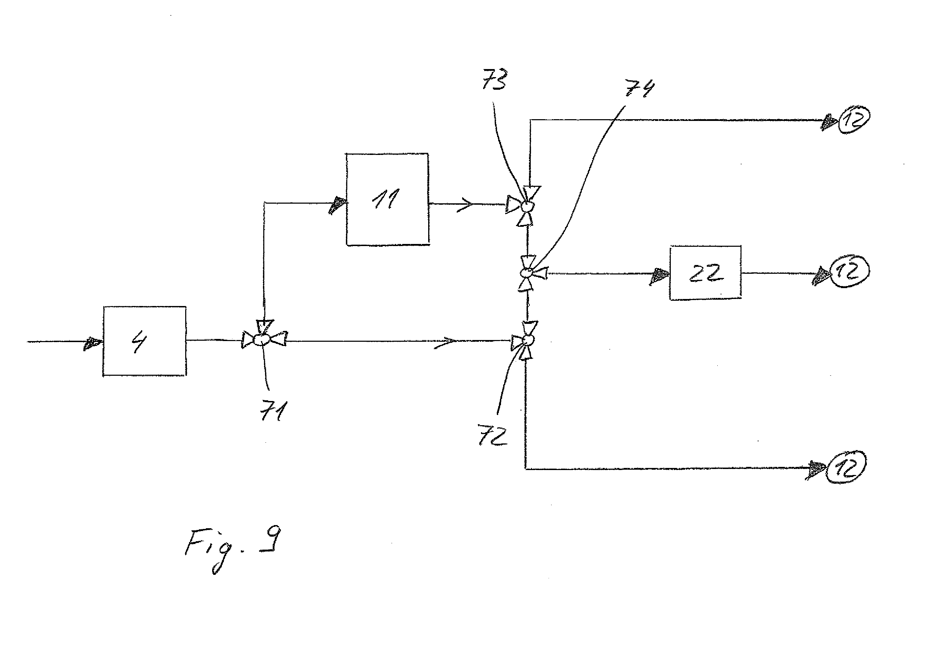

Im Flussdiagramm der Figur 9 sind Variationsmöglichkeiten hinsichtlich der Zerkleinerung von Feststoffbestandteilen dargestellt, wobei der Ultraschall-Zerkleinerer 22 in Abweichung vom Diagramm der Figur 1 in auswählbaren Strömungswegen stromabwärts von entweder dem Zerkleinerer 4 oder dem Homogenisierer 11 angeordnet ist. Entsprechend kann eine tierische Fette enthaltende Fraktion über den Zerkleinerer 4 und wahlweise den Homogenisierer 11 zum Zerkleinerer 22 geführt werden. Ferner kann die Biomasse nach einer Zerkleinerung mittels des Zerkleinerers 4 unter Umgehung des Homogenisierers 11 zum Auslass 12 geführt werden.

Die Biomasse wird nach der Zerkleinerung mittels des Zerkleinerers 4 über eine erste Verzweigung mit einem ersten Steuermittel 71, beispielhaft ein Dreiwegeventil, zum Homogenisierer 11 oder unter Umgeheung des Homogenisierers 11 zu einer zweiten Verzweigung mit einem zweiten Steuermittel 72, beispielhaft ebenfalls ein Dreiwegeventil, gefördert. Gelangt die Biomasse zum Homogenisierer 11, wird sie nach der weiteren Zerkleinerung und Feindispergierung im Homogenisierer 11 über eine dritte Verzweigung mit einem dritten Steuermittel 73, beispielhaft wieder ein Dreiwegeventil, zum Auslass 12 oder über ein viertes Steuermittel 74, das beispielhaft ebenfalls ein Dreiwegeventil sein kann, zum Ultraschall-Zerkleinerer 22 gefördert und dort der beschriebenen Ultraschallzerkleinerung unterzogen. Von dort wird die Biomasse zum Auslass 12 geführt. Bei Umgehung des Homogenisierers 11 wird die Biomasse über die zweite Verzweigung bei 72 entweder unter Umgehung auch des Zerkleinerers 22 oder zwecks Ultraschallbehandlung über die bei 74 gebildete vierte Verzweigung, den Zerkleinerer 22 und den Auslass 12 zu dem Fermenter geführt. Der Homogenisierer 11 und der Zerkleinerer 22 können in einer Variation des Flussdiagramms die Position tauschen.

Die Möglichkeiten der Zuschaltung und Umgehung von wahlweise dem Homogenisierer 11 oder dem Zerkleinerer 22 einschließlich der Möglichkeit beide Komponenten in Serie dazu zu schalten trägt zur Senkung der Energiekosten bei. Enthält die Biomasse in nennenswertem Ausmaß zellulosehaltige Faserbestandteile, wird der Homogenisierer 11 vorzugsweise zugeschaltet, d. h. die Biomasse wird über den Homogenisierer 11 und über den Zerkleinerer 22 oder unter Umgehung des Zerkleinerers 22 zum Auslass 12 gefördert. Enthält die Biomasse in nennenswertem Ausmaß tierische Fette, wird der Zerkleinerer 22 vorzugsweise zugeschaltet, d. h. die Biomasse wird über den Zerkleinerer 22 und über den Homogenisierer 11 oder unter Umgehung des Homogenisierers 11 zum Auslass 12 gefördert. Fehlen fasrige Bestandteile oder tierische Fette, wird oder werden der Homogenisierer 11 oder der Zerkleinerer 22 vorzugsweise umgangen und abgeschaltet.

Das Diagramm der Figur 9 zeigt Möglichkeiten der Modifikation der Vorrichtung. So wird beispielsweise der Zerkleinerer 22 in den Strom der Biomasse integriert. Allerdings kann die Vorrichtung der Figur 1 in einer weiteren Alternative um die Verzweigungen bei 72, 73 und 74 und die Leitungen ergänzt werden, die vom Homogenisierer 11 zum Zerkleinerer 22 und vom Zerkleinerer 4 unter Umgehung des Homogenisierers 11 zum Zerkleinerer 22 oder gänzlich ohne nachgeschaltete Zerkleinerung zum Auslass 12 führen. Das Steuermittel 71 kann das Steuermittel 14 der Figur 1 sein. Das Steuermittel 16 kann beispielsweise zwischen dem Homogenisierer 11 und dem Steuermittel 73 angeordnet sein.

### Bezugszeichen:

- 1: Plattform, Container-Plattform
- 2: Vorgrube
- 3: Einlass für flüssige Biomasse
- 4: Mechanischer Zerkleinerer
- 5: Fördereinrichtung, Pumpe
- 6: Einlass für trockene Biomasse
- 7: Vorlagebehälter
- 7a: Boden
- 8: Mischeinrichtung
- 9: -
- 10: Anmischbehälter
- 11: Homogenisierer
- 12: Auslass für das behandelte Substrat
- 13: Hauptleitung
- 14: Steuermittel, Mehrwegeventil
- 15: Steuermittel, Mehrwegeventil
- 16: Steuermittel, Mehrwegeventil
- 17: Leitung
- 18: Rückführleitung
- 19: Rückführleitung
- 20: Sammelbehälter
- 21: Dosierpumpe
- 22: Ultraschall-Zerkleinerer
- 23: Rohr
- 24: Ultraschallerzeuger
- 25: Wasseranschluss
- 26: Steuermittel, Absperrventil
- 27: Rückschlagventil
- 28: Steuermittel, Absperrventil
- 29: Leitung für Sumpfentleerung
- 30: Viskosimeter
- 31: Steuermittel, Absperrventil
- 32: Steuermittel, Absperrventil
- 33: Steuermittel, Absperrventil
- 34: Steuermittel, Absperrventil
- 35: Düse
- 36: Schauglas
- 37: Entlüftung
- 38: Aufsetzbare Lampe
- 39: Füllstandssensor
- 40: Rotor
- 41: Scherkranz
- 42: Scherkranz
- 43: Scherkranz
- 44: Welle
- 45: Träger
- 46: Schermesser
- 47: Schergegenkante
- 48: -
- 49: -
- 50: Gehäuse
- 51: Scherkranz
- 52: Scherkranz
- 53: Scherkranz
- 54: -
- 55: Träger
- 56: Schermesser
- 57: Scherkante
- 58: Einlass
- 59: Auslass
- 60: Gehäuse
- 61: Einlass
- 62: Auslass
- 63: Strömungsabschnitt
- 64: Messerwelle
- 65: Messerwelle
- 66: Getriebe
- 67: Motor
- 68: -
- 69: -
- 70: Steuermittel, Mehrwegeventil
- 71: Steuermittel, Mehrwegeventil
- 72: Steuermittel, Mehrwegeventil
- 73: Steuermittel, Mehrwegeventil

- A: Zentrum des Rohrquerschnitts
- R: Rotationsachse
- Z: Hochachse
- SPS: Steuerung

## Patentansprüche

1. Vorrichtung zur Erzeugung einer Suspension aus Biomasse, die Vorrichtung umfassend:
a) einen Einlass (3, 6) für die Biomasse,
b) einen Zerkleinerer (4) oder Homogenisierer (11) für die Zerkleinerung der Biomasse,
c) einen stromab von dem Zerkleinerer (4) oder Homogenisierer (11) angeordneten Auslass (12) für die Biomasse,
d) einen Behälter (7, 10) für die Biomasse,
e) eine Fördereinrichtung (4, 5, 8) für die Biomasse,
f) und eine Plattform (1), auf welcher der Einlass (3, 6), der Auslass (12), der Zerkleinerer (4) oder Homogenisierer (11), der Behälter (7, 10) und die Fördereinrichtung (4, 5, 8) angeordnet sind,
g) wobei die Vorrichtung als Einheit auf der Straße oder Schiene transportabel ist.

2. Vorrichtung nach Anspruch 1, **dadurch gekennzeichnet, dass** die Plattform (1) einen Container bildet, der vorzugsweise begehbar ist, und dass wenigstens eine der Komponenten aus der Gruppe bestehend aus dem Zerkleinerer (4) oder Homogenisierer (11), der Fördereinrichtung (4, 5, 8) und dem Behälter (7, 10) in dem Container angeordnet ist.

3. Vorrichtung nach einem der vorhergehenden Ansprüche, **dadurch gekennzeichnet, dass** die Vorrichtung über alles eine Breite (B) von höchstens 2,5 m oder Höhe (H) von höchstens 4 m aufweist.

4. Vorrichtung nach einem der vorhergehenden Ansprüche, **dadurch gekennzeichnet, dass** sie wenigstens eines der folgenden Merkmale aufweist:
- eine Wand des Containers (1) nach Anspruch 2 bildet auch eine Wand des Behälters (7),
- in dem Behälter (7) ist nahe bei einem Boden des Behälters (7) eine Austragseinrichtung für die in dem Behälter (7) enthaltene Biomasse angeordnet,
- der Behälter (7) ist durch den Einlass (6) der Vorrichtung von oben mit der Biomasse befüllbar,
- der Behälter (10) enthält flüssige Biomasse und feste Partikel der Biomasse.

5. Vorrichtung nach einem der vorhergehenden Ansprüche, **dadurch gekennzeichnet, dass** der Einlass (3) für fließfähige, vorzugsweise flüssige Biomasse vorgesehen ist und die Vorrichtung einen weiteren Einlass (6) für zumindest im Wesentlichen trockene Biomasse und eine vorzugsweise auf der Plattform (1) angeordnete Mischeinrichtung (8, 10) zum Vermischen der fließfähigen und der trockenen Biomasse umfasst.

6. Vorrichtung nach dem vorhergehenden Anspruch, **dadurch gekennzeichnet, dass** sie wenigstens eines der folgenden Merkmale aufweist:
- die Mischeinrichtung (8, 10) ist stromab von dem Zerkleinerer (4) angeordnet,
- die Mischeinrichtung (8) ist zusätzlich zu dem Behälter (10) vorgesehen und stromauf von dem Behälter (10) angeordnet,
- ein Auslass des Behälters (7) ist oberhalb eines Einlasses der Mischeinrichtung (8, 10) angeordnet, so dass die in dem Behälter (7) enthaltene Biomasse von dem Auslass des Behälters (7) in den Einlass der Mischeinrichtung (8, 10) fallen kann,
- der Behälter (10) bildet die Mischeinrichtung oder ist Bestandteil der Mischeinrichtung (8, 10),
- der Auslass (12) ist stromab von dem Behälter (10) oder an dem Behälter angeordnet.

7. Vorrichtung nach einem der vorhergehenden Ansprüche, **dadurch gekennzeichnet, dass** der Behälter (7) einen Vorlagebehälter für zumindest im Wesentlichen aus festen Bestandteilen bestehende Biomasse bildet und die Vorrichtung eine vorzugsweise auf der Plattform (1) angeordnete Mischeinrichtung (8, 10) umfasst zum Vermischen der festen Bestandteile aus dem Behälter (7) mit fließfähiger Biomasse, die durch den Einlass (3) zuführbar ist, oder mit Wasser, das durch einen auf der Plattform (1) angeordneten Wasseranschluss (25) zuführbar ist.

8. Vorrichtung nach einem der vorhergehenden Ansprüche, **dadurch gekennzeichnet, dass** der Zerkleinerer (4) und der Homogenisierer (11) auf der Plattform (1) angeordnet sind und wenigstens eines der folgenden Merkmale erfüllt ist:
- der Homogenisierer (11) ist für eine im Vergleich zu dem Zerkleinerer (4) feinere Zerkleinerung von Biomasse ausgebildet und stromab von dem Zerkleinerer (4) angeordnet,
- der Zerkleinerer (4) ist für eine Zerkleinerung von in der Biomasse enthaltenen Partikeln auf eine größte Erstreckung von maximal 50 mm, vorzugsweise maximal 30 mm, ausgebildet,
- der Homogenisierer (11) ist für eine Zerkleinerung von in der Biomasse enthaltenen Partikeln auf eine maximale Erstreckung von 4 mm, vorzugsweise maximal 2 mm, ausgebildet,
- der Zerkleinerer (4) wenigstens eines aus Brechwerk, Reißwerk und Schneidwerk und der Homogenisierer ein mehrstufiges Scherwerk, vorzugsweise Dispergierwerk, aufweist,
- der Zerkleinerer (4) weist drehantreibbare Messerwellen auf mit jeweils mehreren um die jeweilige Wellenachse umlaufenden und axial voneinander beabstandete Reihen von Schneidzähnen, wobei die Messerwellen zueinander außenachsig angeordnet sind und ineinander greifen, so dass durch einen Spalt zwischen den Messerwellen geförderte Biomasse durch wenigstens eines aus Schneiden, Reißen und Drücken zerkleinert wird,
- eine erste Leitung (13) führt von dem Zerkleinerer (4) in den Behälter (10), und der Homogenisierer (11) ist in einer zweiten Leitung (17) angeordnet, die stromab von dem Zerkleinerer (4) und stromauf von dem Behälter (10) von der ersten Leitung (13) abzweigt.

9. Vorrichtung nach einem der vorhergehenden Ansprüche, **dadurch gekennzeichnet, dass** die Vorrichtung den Homogenisierer (11) umfasst und wenigstens eines der folgenden Merkmale erfüllt ist:
- der Homogenisierer (11) weist ein mehrstufiges Scherwerk mit ersten Scherkränzen (41, 42, 43) und zweiten Scherkränzen auf, wobei die ersten Scherkränze (41, 42, 43) relativ zu den zweiten Scherkränzen um eine Drehachse (R) drehbar sind und jeweils einen der zweiten Scherkränze (51, 52, 53) umgeben oder von einem der zweiten Scherkränze (51, 52, 53) umgeben werden,
- der Homogenisierer (11) ist in einem durch den Behälter (10) und den Homogenisierer (11) führbaren Strom der Biomasse näher bei dem Auslass (12) angeordnet als der Behälter (10),
- die mittels des Homogenisierers (11) fein zerkleinerte Biomasse ist in den Behälter (10) führbar,
- die mittels des Homogenisierers (11) fein zerkleinerte Biomasse ist wahlweise zu dem Auslass (12) oder in den Behälter (10) führbar.

10. Vorrichtung nach einem der vorhergehenden Ansprüche, **dadurch gekennzeichnet, dass** auf der Plattform (1) ein weiterer Zerkleinerer (22), vorzugsweise Ultraschall-Zerkleinerer, für tierische Fette enthaltende Biomasse angeordnet und die tierische Fette enthaltende Biomasse über den weiteren Zerkleinerer (22) vorzugsweise unter Umgehung des Behälters (10) zu dem Auslass (12) führbar ist.

11. Vorrichtung nach einem der vorhergehenden Ansprüche, **dadurch gekennzeichnet, dass** auf der Plattform (1) ein Wasseranschluss (25) für die Einspeisung von Wasser, vorzugsweise in den Behälter (10) oder Homogenisierer (11), oder für eine Spülung von Komponenten (14-16, 26, 31-34), vorzugsweise Steuermittel, der Vorrichtung angeordnet ist.

12. Vorrichtung nach einem der vorhergehenden Ansprüche, **dadurch gekennzeichnet, dass** auf der Plattform (1) eine Einrichtung (5, 30) zur Ermittlung der Viskosität eines Stroms von mittels der Vorrichtung behandelter Biomasse angeordnet ist und dass die Vorrichtung eine Steuerung umfasst, die in Abhängigkeit von der ermittelten Viskosität die Fördereinrichtung (5) oder ein auf der Plattform (1) angeordnetes Steuermittel (14-16, 26, 31-34), vorzugsweise ein Absperrventil oder Mehrwegeventil, ansteuert, über das dem Strom der Biomasse Flüssigkeit beimischbar ist.

13. Vorrichtung nach einem der vorhergehenden Ansprüche, **dadurch gekennzeichnet, dass**
- der Homogenisierer (11) einen um eine Rotationsachse (R) drehangetriebenen ersten Scherkranz (41, 42, 43) und einen zweiten Scherkranz (51, 52, 53) aufweist, wobei vorzugsweise einer der Scherkränze den anderen umgibt,
- der erste Scherkranz (41, 42, 43) um die Rotationsachse (R) angeordnete, quer zu einer Umfangsrichtung des ersten Scherkranzes (41, 42, 43) weisende Scherkanten (47) und der zweite Scherkranz (51, 52, 53) um die Rotationsachse (R) angeordnete, quer zu der Umfangsrichtung weisende Schergegenkanten (57) aufweist
- und die Biomasse in einen zwischen den Scherkränzen um die Rotationsachse (R) verlaufenden Spalt förderbar ist, so dass sie bei einem Drehantrieb des ersten Scherkranzes (41, 42, 43) mittels der miteinander scherenden Scher- und Schergegenkanten (47, 57) zerkleinert wird.

14. Vorrichtung nach dem vorhergehenden Anspruch, **dadurch gekennzeichnet, dass** der Homogenisierer (11) einen vorzugsweise drehangetriebenen dritten Scherkranz (41, 42, 43) mit um die Rotationsachse (R) angeordneten, quer zu einer Umfangsrichtung des dritten Scherkranzes weisenden Scherkanten (47) umfasst und die Biomasse aus dem Spalt in einen zwischen dem zweiten und dem dritten Scherkranz um die Rotationsachse (R) verlaufenden weiteren Spalt förderbar ist, so dass sie bei einem Drehantrieb des ersten Scherkranzes (41, 42, 43) auch mittels der miteinander scherenden Scher- und Schergegenkanten (47, 57) des zweiten und des dritten Scherkranzes zerkleinert wird.

15. Vorrichtung nach dem vorhergehenden Anspruch, **dadurch gekennzeichnet, dass** der Homogenisierer (11) einen vierten Scherkranz (51, 52, 53) mit um die Rotationsachse (R) angeordneten, quer zu der Umfangsrichtung weisenden Schergegenkanten (57) umfasst, wobei der dritte und der vierte Scherkranz vorzugsweise einander umgeben, und dass die Biomasse aus dem zwischen dem ersten und dem zweiten Scherkranz gebildeten Spalt in einen zwischen dem dritten und dem vierten Scherkranz um die Rotationsachse (R) verlaufenden weiteren Spalt förderbar ist, so dass sie bei einem Drehantrieb des ersten und vorzugsweise des dritten Scherkranzes (41, 42, 43) auch mittels der miteinander scherenden Scher- und Schergegenkanten (47, 57) des dritten und des vierten Scherkranzes zerkleinert wird.

16. Vorrichtung nach dem vorhergehenden Anspruch, **dadurch gekennzeichnet, dass** der dritte und der vierte Scherkranz den ersten und den zweiten Scherkranz umgeben oder von dem ersten und dem zweiten Scherkranz umgeben werden, so dass die Scherkränze radial hintereinander drei jeweils um die Rotationsachse (R) verlaufende Spalte bilden, und dass die Biomasse sukzessive in radialer Richtung durch diese Spalte förderbar ist, so dass sie bei einem Drehantrieb des ersten und vorzugsweise des dritten Scherkranzes (41, 42, 43) in jedem der Spalte mittels der miteinander scherenden Scher- und Schergegenkanten (47, 57) der radial jeweils nächstbenachbarten Scherkränze zerkleinert wird.

17. Vorrichtung nach Anspruch 15, **dadurch gekennzeichnet, dass** der erste und der zweite Scherkranz axial vor dem dritten und dem vierten Scherkranz angeordnet sind und die Biomasse sukzessive durch die Spalte förderbar ist, so dass sie bei einem Drehantrieb des ersten und des dritten Scherkranzes (41, 42, 43) in den Spalten mittels der jeweils miteinander scherenden Scherkanten (47) und Schergegenkanten (57) zerkleinert wird.

18. Vorrichtung nach einem der fünf vorhergehenden Ansprüche, **dadurch gekennzeichnet, dass** wenigstens einer der Scherkränze zwischen in Umfangsrichtung benachbarten Scherkanten oder Schergegenkanten radial durchlässig ist, um die Biomasse in radialer Richtung in den mittels des wenigstens einen der Scherkränze gebildeten Spalt zu fördern.

19. Vorrichtung nach einem der vorhergehenden Ansprüche, **gekennzeichnet durch** die Verwendung zur Aufbereitung von Biomasse für einen Fermenter einer Biogasanlage für die Erzeugung methanhaltigen Biogases aus land- oder forstwirtschaftlichen Produkten oder organischem Abfallmaterial.

20. Verfahren für die Fertigung von Vorrichtungen nach einem der vorhergehenden Ansprüche, bei dem eine erste der Vorrichtungen produziert und zertifiziert wird und nach der Zertifizierung der ersten der Vorrichtungen weitere Vorrichtungen hergestellt werden, die der ersten der Vorrichtungen zumindest soweit entsprechen, dass sich die Zertifizierung der ersten der Vorrichtungen auf die weiteren Vorrichtungen erstreckt.
